# EUROPÄISCHE PATENTSCHRIFT

(11) **EP 1 090 596 B1**
(45) Veröffentlichungstag und Bekanntmachung des Hinweises auf die Patenterteilung: **05.07.2006**
(21) Anmeldenummer: 00810689.0
(22) Anmeldetag: 31.07.2000
(51) Int. Cl.: A61B 17/72

(54) **Marknagel für den Humerus**
Intramedullary nail for the humerus
Clou intramédullaire pour l'humerus

(30) Priorität: 30.08.1999 EP 99810778
(43) Veröffentlichungstag der Anmeldung: 11.04.2001
(73) Patentinhaber: Zimmer GmbH, 8404 Winterthur (CH)
(72) Erfinder: Bartsch, Eric, 6340 Baar (CH); Bürgi, Maja, 8505 Pfyn (CH)
(74) Vertreter: Manitz, Finsterwald & Partner GbR

(56) Entgegenhaltungen:
- EP-A- 0 517 435
- EP-A- 0 738 502
- EP-A- 0 882 431
- US-A- 2 998 007

## Beschreibung

Die Erfindung handelt von einem Marknagel vorzugsweise für den Humerus mit den Merkmalen des Oberbegriffs des Anspruches 1.

Ein derartingen Marknagel ist in EP 0 517 435 A1 beschrieben. Aus US 2,998,007 ist ein ähnlichen Gegenstand zur Fitierung von Knochenfrahmen bekannt.

Die Marknagelung an sich ist eine schonende Methode zur operativen Behandlung von Frakturen langer Röhrenknochen. Wichtige Vorteile bestehen in einem sehr kleinen Zugang ohne nennenswerte Narbenbildung und in einer geschlossenen Reposition der Fraktur. Um eine stabile und rotationssichere intramedulläre Frakturschienung zu erhalten, werden Marknägel an beiden Enden mittels Stiften oder Schrauben im Knochen verriegelt.

Bei der Behandlung von Oberarmbrüchen wird diese Verriegelung an der Einführungsstelle (insertionsnah) mit Hilfe eines Zielbügels durchgeführt, der am Ende des Nagels befestigt ist und gleichzeitig als Handgriff zur besseren Einführbarkeit des Marknagels dient. In ihm sind Führungen für den Bohrer vorgesehen, der somit die im Implantat vorhandenen Löcher von aussen exakt treffen kann. Ein Problem bei fast allen Humerus-Marknägeln besteht wegen ihrer geringen Durchmesser bei einer Verriegelung am insertionsfernen Ende. Da ein Auffinden von entfernten Implantatlöchern mit einem langen Zielgerät sehr umständlich ist und sich der Nagel über die Strecke des gesamten Humerus verbiegt und verwindet, erfolgt die insertionsferne Verriegelung Freihand unter Röntgenbildverstärker-Durchleuchtung. Dabei muss ein vorhandenes, ca. 4 mm grosses Loch im Implantat von aussen gesucht, und mit dem Bohrer getroffen werden, was mit Strahlenbelastung und einem hohen Zeitaufwand einhergeht.

Marknägel mit radial ausfahrbaren Verankerungselementen in der Nähe der Eintrittsspitze sind in der Patentschrift U.S. 5,810,820 gezeigt. Radial austretende Drahtspitzen bohren sich in das Knochenmaterial und geben Halt in axialer Richtung. Die mit Spitzen versehenen Drähte haben eine zylindrische Form und sind im eingezogenen Zustand jeweils in Führungen gelagert. Das Prinzip des radialen Austritts beruht darauf, dass die Drähte durch achsiales Zustellen derart in eine radiale Umlenkung gepresst werden, dass sie sich plastisch verformen. Durch axiales Rückstellen werden die Drähte wieder plastisch in eine zylindrische Form zurückverformt. Diese Anordnung hat den Nachteil, dass der Draht zur Richtungsänderung jeweils plastisch verformt werden muss. Eine Funktionskontrolle vor dem Einsetzen des Marknagels birgt die Gefahr in sich, dass sich die Drähte durch die plastische Deformation aufhärten und spröder werden. Ein Weglassen der Funktionskontrolle ist auch nicht sehr sinnvoll. Im weiteren müssen die Drähte innerhalb des Marknagels sehr genau allseitig geführt werden, damit kein Knicken stattfindet.

Bei einem Marknagel für den Humerus kommt erschwerend hinzu, dass der Durchmesser des Markraums klein bemessen ist und nur Marknägel mit kleinen Durchmessern zulässt. Individuelle, den Draht voll umschliessende Führungen sind nur noch mit grossem Aufwand herstellbar. Es ist daher Aufgabe der Erfindung, einen einfach konzipierten Humerus-Marknagel aufzuzeigen.

Diese Aufgabe wird mit den Kennzeichen des unabhängigen Anspruchs 1 gelöst.

Diese Anordnung hat den Vorteil, dass die Arbeit für das radiale Eindringen in Knochenmaterial mit dem Zurückziehen der Kralle in den Schaft des Marknagels bereits gespeichert ist. Eine Funktionskontrolle ist jederzeit möglich, da die Drähte nur im elastischen Bereich deformiert werden. Die abhängigen Ansprüche 2 bis 10 sind vorteilhafte Weiterbildungen der Erfindung. Für den einzelnen Draht genügt eine offene, zylindrische Führungsrinne, welche die Drahtspitze führt und durch einen radialen Durchbruch unterbrochen ist. Die Führungsrinnen lassen sich auch bei kleinen Schaftabmessungen durch Stossen herstellen, wenn die Spitze des Marknagels später aufsetzbar ist. Die radialen Durchbrüche verjüngen sich zur Eintrittsspitze des Marknagels hin und zentrieren die Drähte. Gleichzeitig findet eine seitliche Abstützung an den radial austretenden Drähten statt. Durch die Führungsrinnen entsteht an den Drahtspitzen bereits eine Verdrehsicherung. Zusätzlich können Nocken am Fuss der Kralle angebracht sein, die als zusätzliche Verdrehsicherung in den Führungsrinnen laufen. Im Fuss der Kralle ist eine Stellschraube drehbar gelagert, welche jedoch in axialer Richtung an der Kralle fixiert ist. Durch das Zusammenfassen der Drähte in einer Kralle, kann die Lage der vorgebogenen Drähte zueinander vorgegeben werden. Anstatt die Ebenen, in denen die Drähte abgebogen sind, konzentrisch durch die Längsachse laufen zu lassen, wird mehr Raum für die elastische Deformation der Drähte gewonnen, wenn diese Ebenen einen Abstand zur Längsachse aufweisen. Querbohrungen, die in der Nähe des Befestigungskopfes liegen, können trotz der kleinen Durchmesser für einen Humerus-Marknagel mit einem Zielbügel angebracht werden, da dessen Armlänge sehr kurz bemessen ist. Wenn die Stellschraube von der Seite der Spitze des Marknagels einbringbar ist, kann der hohle Marknagel im Bereich zwischen Stellschraube und Befestigungskopf eine Krümmung aufweisen. In einem solchen Fall ist ein biegbares Schraubwerkzeug notwendig, um die Stellschraube zu verstellen. Der vorliegende Marknagel ist nicht auf den Humerus beschränkt und kann grundsätzlich auch bei anderen Röhrenknochen eingesetzt werden.

Eine weitere Verbesserung, die ganz allgemein für Marknägel ihre Anwendung findet, besteht darin, einen Marknagel in mindestens einer vorgegebenen Zone seines Schaftes biegeelastisch zu gestalten, damit er einer Krümmung des Markkanals folgen kann. Dabei versteht es sich, dass diese biegeelastische Zone nicht in der Nähe einer Bruchstelle eines Röhrenknochens liegen sollte. Besondere Vorteile ergeben sich, wenn diese biegeelastische Zone auch noch als Zugfeder verwendbar ist, weil sie beispielsweise als ein- oder mehrgängige Wendel ausgeführt ist, deren Windungen im spannungslosen Zustand einen geringen vorgegebenen Abstand aufweisen, derart, dass bei einem vollständigen Zusammenpressen dieser Schraubenfeder deren Windungen nur elastisch deformiert sind, um das Einschlagen des Marknagels zu erleichtern. Nach dem Verankern eines ersten Endes des Marknagels kann vom entgegengesetzten Ende des Marknagels Zug aufgebracht werden, welcher diese Schraubenfeder in Zugrichtung spannt, und anschliessend unter Beibehaltung der Zugkraft an diesem entgegengesetzten Ende des Marknagels eine weitere Verankerung angebracht werden. Die Schraubenfeder als Zugfeder übt dann über die beidseitigen Verankerungen des Marknagels eine Kompression auf den Knochen resp. auf eine dazwischenliegende Bruchstelle aus, wobei sich die Kompression wegen der im Vergleich zum starren Marknagel viel weicheren Federwirkung der Zugfeder nicht so schnell abbauen kann, da eine geringe Änderung des Federweges nur eine geringe Änderung der Kompressionskraft verursacht. Die Einführung einer solchen biegeelastischen Zone ist überall dort von besonderem Vorteil, wo die Verankerung der beiden Enden eines Marknagels unabhängig von der Lage dieser beiden Enden zueinander erfolgen kann. Bei einem hohlen Marknagel wie er in den nachfolgenden Ausführungsbeispielen aufgezeigt ist, wird die Verankerung der beiden Enden des hohlen Marknagels unabhängig voneinander von dem Ende des Befestigungskopfes aus vorgenommen. Die Biege- und Zugfeder ist daher als Wendel aus dem hohlen Schaft geschnitten, um einem biegsamen Schraubendreher auf der Innenseite Platz zu lassen. Eine Ausführung eines vollen Marknagels mit einer Biege und/oder Zugfeder in einer vorgegebenen Zone ist ebenfalls sinnvoll, wenn die Verankerung der beiden Enden unabhängig von ihrer Position zueinander erfolgt. In der Patentanmeldung WO 98/02104 ist ein voller, durch ein Bohrstück verlängerter Marknagel gezeigt, an dessen beiden Enden unabhängig voneinander eine Zielvorrichtung aufsetzbar ist, um das jeweilige Ende zu verankern. Auch hier hat eine im Mittelteil zwischen den Querbohrungen liegende biegeelastische und eventuell auch noch zugelastische Zone keinen Einfluss auf das Anfahren der Querbohrungen. Wenn eine solche biege- und eventuell zugelastische Zone geplant ist, sind Biegeauslenkungen von mehr als 2° sinnvoll. Bei starren gekrümmten Marknägeln sind Biegezonen mit einer Biegeauslenkung von 4° bis 9° üblich. In diesem Bereich sollten auch biegeelastische Marknägel auslenkbar sein. Wie eine solche biegeelastische Zone aussehen kann, wird in der EP-B-0 614 020 für ein flexibles Schraubwerkzeug beschrieben und ist auf einen Marknagel übertragbar, wenn der Aussendurchmesser der biegeelastischen Zone nicht grösser als der Aussendurchmesser des starren Teils ist. Grundsätzlich sind alle Formen von biegeelastischen Zugfedern möglich, solange sie nicht über den Schaftdurchmesser vorstehen.

Im folgenden wird die Erfindung anhand von Ausführungsbeispielen beschrieben. Es zeigen:
- Fig. 1:: Schematisch eine vergrösserte Seitenansicht eines erfindungsgemässen Marknagels;
- Fig. 2: schematisch den Innenteil des Marknagels von Fig. 1;
- Fig. 3: schematisch den Marknagel von Fig. 1 mit eingezogener Kralle;
- Fig. 4: schematisch eine starke Vergrösserung eines erfindungsgemässen Marknagels im Bereich der Führungsrinnen;
- Fig. 5: schematisch einen Schnitt im oberen Bereich der radialen Durchbrüche von Fig. 4;
- Fig. 6: schematisch einen Schnitt im unteren Bereich der radialen Durchbrüche von Fig. 4;
- Fig. 7: schematisch eine Anordnung wie in Fig. 6, bei der die Drähte mit ihren Krümmungsebenen einen Abstand zur Längsachse aufweisen;
- Fig. 8: schematisch einen Marknagel mit Zielbügel und mit einer Krümmung oberhalb der Querbohrungen; und
- Fig. 9: schematisch einen Marknagel analog zu Fig. 8, bei dem die Krümmung durch eine biege- und zugelastische Zone ersetzt ist.

In den Figuren ist ein Marknagel vorzugsweise für den Humerus gezeigt mit einem Befestigungskopf für eine Einschlag- und Zielvorrichtung, wobei der Marknagel mindestens eine Querbohrung anschliessend an den Befestigungskopf aufweist, und mit einer Eintrittsspitze, in deren Nähe radial Verankerungselemente ausfahrbar sind. Die Verankerungselemente sind in einer Kralle aus gekrümmten Drähten zusammengefasst, wobei die Drähte in einem nicht ausgefahrenen Zustand innerhalb des Marknagels elastisch deformiert sind und mit einer axialen Zustellung der Kralle radial herausspringen und ihre ursprüngliche gekrümmte Form annehmen.

In den nachfolgenden Figuren sind die Hinweiszeichen gleich verwendet.

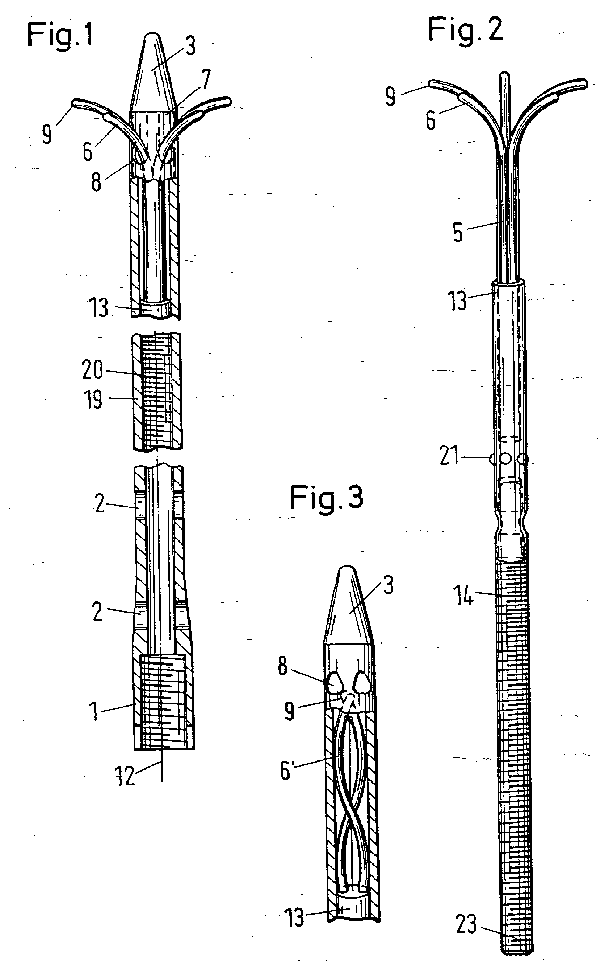

In Figur 1 und 2 besitzt ein hohler Marknagel einen Befestigungskopf 1, an welchen Querbohrungen 2 anschliessen und einen Schaft 19 mit einer nachträglich montierten Spitze 3. Unterhalb der Spitze 3 sind radiale Durchbrüche 8 angebracht, aus denen gekrümmte Drähte 6 austreten, die in einer Kralle 5 zusammengefasst sind. Die Drähte 6 der Kralle 5 sind in einem Fuss 13 fest verankert und sind in einer Krümmung radial abgebogen. Im Fuss 13 ist eine Stellschraube 14 drehbar gelagert, jedoch in axialer Richtung fixiert. Zur Stellschraube 14 besteht ein Gegengewinde 20 im Schaft 19. Der Innenteil (Figur 2) wird bei abgenommener Spitze 3 von oben eingeschoben bis die Stellschraube 14 am Gewinde 20 ansteht und mit einem Schraubwerkzeug von der Gegenseite an ihrem Innensechskant 23 weiter hineinbewegt wird. Die Kralle 5 wird soweit in den Schaft 19 hineingezogen, bis die Drähte 6 vollständig verschwunden sind und mit ihren Spitzen 9 die radialen Durchbrüche 8 überfahren haben. Die Drähte 6 sind so dimensioniert, dass sie durch ihre Streckung nur elastisch deformiert werden. Auf der Innenseite des Schaftes 19 sind Führungsrinnen 7 in Längsrichtung angebracht, die sich mit den Durchbrüchen 8 decken und die Drahtspitzen 9 führen und gegen Verdrehung sichern. Eine zusätzliche Drehsicherung ist durch Nocken 21 am Fuss 13 der Kralle möglich, wenn die Nocken 21 in die Führungsrinnen 7 eingreifen. Durch Heraus- und Hineinschrauben der Stellschraube 14 kann die Kralle auf und ab bewegt werden. Sobald die Krallenspitzen 9 von unten kommend die Durchbrüche 8 erreichen, springen sie radial nach aussen und bohren sich in einen sie umgebenden Röhrenknochen, falls der Marknagel in diesen eingeschlagen ist. Durch ein Fortsetzen der Axialbewegung wird der gestreckte Teil vollständig zur selbständigen Krümmung freigegeben.

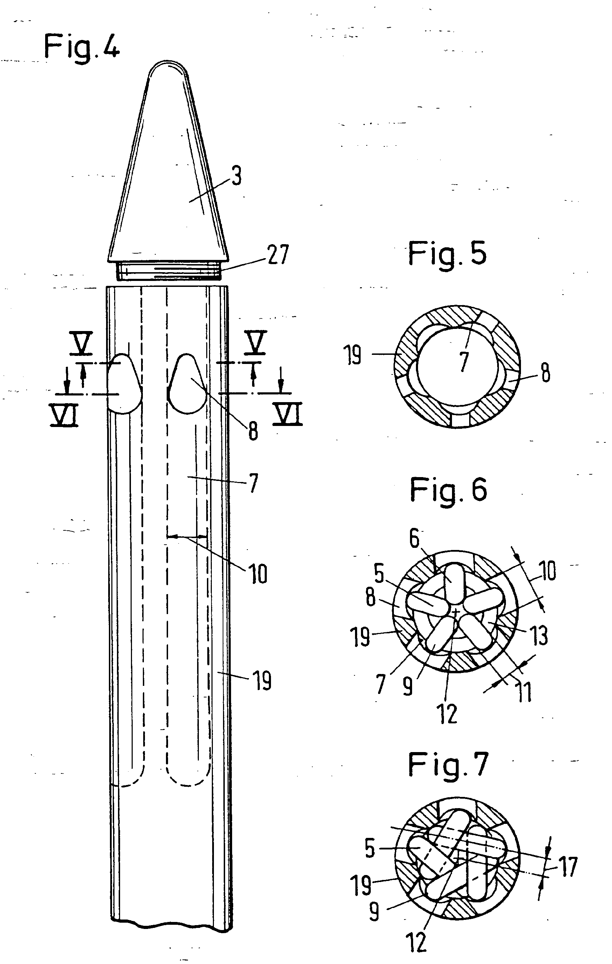

Am Beispiel der Figuren 4, 5 und 6 wird der Aufbau genauer erklärt. Der Schaft 19 des Marknagels hat einen Aussendurchmesser von 6 mm und besteht aus einem Metallrohr, beispielsweise Stahl oder Titan, auf welches eine Spitze 3 mit Gewinde 27 aufschraubbar ist. Auf der Innenseite dieses Rohrs sind fünf Führungsrinnen 7 mit einer möglichst grossen Breite 10 gestossen, um die Spitzen 9 der Kralle 5 zu führen und elastisch deformiert zu halten. Die Drähte 6 der Kralle 5 sind aus dem Fuss 13 zunächst zylindrisch und parallel zur Längsachse 12 herausgeführt und im spannungsfreien Zustand mit einem Krümmungsradius von 8 mm radial nach aussen um etwa 90° gekrümmt. Bei der Montage der Kralle 5 werden die Krallenspitzen 9 zwangsläufig in die Führungsrinnen 7 eingezogen und elastisch vorgespannt. Wegen der Krümmung der Drähte 6 kann die Kralle 5 mit ihren Spitzen 9 die radialen Durchbrüche 8 auf dem Weg nach innen überfahren. Wird jedoch die Kralle nach aussen verschoben, dann werden die vorgespannten Spitzen 9 zwangsläufig von den Durchbrüchen 8, welche eine grösste Breite 10 der Führungsrinnen 7 aufweisen, eingefangen und - weil sich die Durchbrüche 8 zur Spitze 3 des Marknagels verjüngen - zentriert und seitlich abgestützt. Dabei bewegen sich die Drähte 6 in dem Mass, wie sie in axialer Richtung freigegeben werden, radial nach aussen.

In Figur 6 erkennt man, dass die Ebenen der Drahtkrümmung konzentrisch auf die Längsachse 12 ausgerichtet sind und relativ wenig Raum für die Streckung der Drähte zugestehen.

In Figur 7 ist daher eine Anordnung gezeigt, bei der die Ebenen der Drahtkrümmung um einen Abstand 17 von der Längsachse 12 versetzt an der Längsachse vorbeilaufen und einen wesentlich grösseren Raum bei der Streckung der Drähte 6 zu Verfügung stellen. Der Draht darf in diesem Fall dicker und steifer als in Figur 6 sein und wird immer noch in seinem elastischen Bereich deformiert. Da sich der Draht nur mit seiner Spitze 9 selber führt, kann er mit seinem zylindrischen Teil, der im Fuss 13 abgestützt wird, so lang ausgeführt werden, dass ein Teil der Krümmung beim eingezogenen Draht durch Krümmung des zylindrischen Teils in Gegenrichtung aufgenommen wird (siehe Figur 3). Der gestreckte Draht 6' erhält dann eine S-förmige Krümmung.

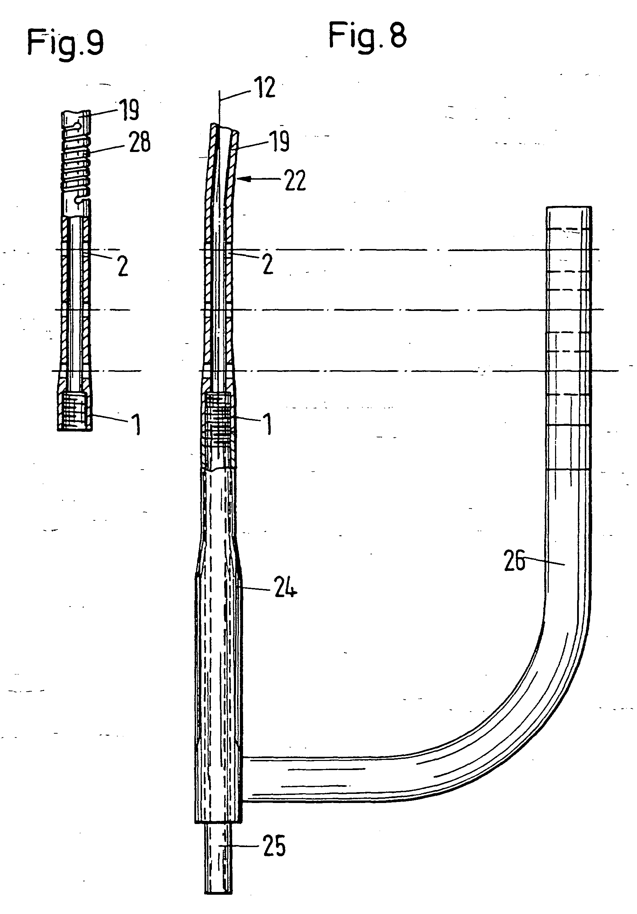

In Figur 8 erweitert sich der Schaft 19 des Marknagels zu einem Befestigungskopf 1. Eine in einer vorgegebenen Position aufsteckbare Einschlag- und Zielvorrichtung ist mit einer hohlen Befestigungsschraube 25 am Kopf 1 angeschraubt und ermöglicht es über einen parallel zum Schaft geführten Zielbügel 26, Querbohrungen 2 im eingeschlagenen Schaft 19 aufzufinden und mit Verankerungsschrauben zu versehen, um zu der Verankerung durch die Kralle 5 an der Spitze 3 des Marknagels eine zweite Verankerung am Befestigungskopf 1 zu erreichen. Dabei darf der Marknagel auch eine leichte Krümmung 22 zur Längsachse 12 des Befestigungskopfes 1 aufweisen, wenn der Schraubendreher für die Stellschraube 14 des Innenteils (Figur 2) in Biegerichtung flexibel ist. Der implantierte Marknagel erhält eine Verschlusskappe (hier nicht gezeigt), die den Innenraum von einwachsendem Gewebe freihält. Ebenso sind die Querbohrungen 2 durch Knochenschrauben weitgehend verschlossen. Zum Entfernen des Marknagels müssen Verschlusskappe und Knochenschrauben in den Querbohrungen entfernt werden. Anschliessend kann ein Schraubwerkzeug in den Innensechskant 23 (Figur 2) eingebracht werden, um die Kralle 5 in den Marknagel zurückzuziehen. Daran anschliessend kann ein Gleithammer (hier nicht gezeigt) am Befestigungskopf angeschraubt werden, um den Marknagel auszuschlagen.

In Figur 9 ist der hohle Schaft 19 durch eine Nut in Form einer Wendel mit einer biege- und zugelastischen Zone 28 versehen. Eine solche Nut kann durch Wasserstrahlschneiden, funkenerosives Drahtschneiden, Laserbearbeitung oder Abwälzfräsen mit einem Scheibenfräser hergestellt werden. Der Marknagel wird mit einer Einschlag- und Zielvorrichtung gemäss Figur 8 eingeschlagen und nimmt die Krümmung des Markraumes an. In einem nächsten Schritt wird mit einem flexiblen Schraubendreher die Kralle 5 ausgefahren und im Knochen verankert. In einem weiteren Schritt wird der Befestigungskopf 1 mit dem Fuss der Zielvorrichtung soweit gegenüber dem Knochen auf der Insertionsseite herausgezogen, bis eine vorgesehene Zugspannung an der elastischen Zone 28 eintritt. Diese Stellung wird gehalten bis mit dem Zielbügel im Knochenmaterial Verlängerungen zu den Querbohrungen 2 gebohrt sind und Knochenschrauben zur Verankerung des Endes mit dem Befestigungskopf 1 eingeschraubt sind.

## Patentansprüche

1. Marknagel, vorzugsweise für den Humerus, mit einem Befestigungskopf (1) für eine Einschlag- und Zielvorrichtung (24), wobei der Marknagel mindestens eine Querbohrung (2) anschliessend an den Befestigungskopf (1) aufweist, und mit einer Eintrittsspitze (3) sowie mit in deren Nähe radial ausfahrbaren Verankerungselementen (4), wobei die Verankerungselemente (4) in einer Kralle (5) aus gekrümmten Drähten (6) zusammengefasst sind, wobei die Drähte (6) in einem nicht ausgefahrenen Zustand innerhalb des Marknagels elastisch deformiert sind und mit einer axialen Zustellung der Kralle radial herausspringen und ihre ursprüngliche gekrümmte Form annehmen, und wobei auf der Innenseite des Marknagels in Längsrichtung verlaufende Führungsrinnen (7) angebracht sind, die jeweils einen radialen Durchbruch (8) aufweisen, um die Spitzen (9) der deformierten Drähte (6') bis zu ihrem radialen Austritt zu führen,
**dadurch gekennzeichnet, dass** die Durchbrüche die Breite (10) der Führungsrinne (7) haben und sich in Richtung der Eintrittsspitze (3) bis auf die Breite (11) eines Drahtes (6) verjüngen, um den axial zugestellten und radial ausgetretenen Drähten (6) in axialer Richtung und gegen Drehung um die Längsachse (12) Unterstützung zu geben.

2. Marknagel nach Anspruch 1, **dadurch gekennzeichnet, dass** die Kralle (5) einen Fuss (13) aufweist, welcher über eine Stellschraube (14) axial verschiebbar ist.

3. Marknagel nach Anspruch 1 oder 2, **dadurch gekennzeichnet, dass** an der Kralle eine Verdrehsicherung angebracht ist.

4. Marknagel nach einem der Ansprüche 1 bis 3, **dadurch gekennzeichnet, dass** mindestens drei gekrümmte Drähte (6) die Kralle (5) bilden.

5. Marknagel nach einem der Ansprüche 1 bis 4, **dadurch gekennzeichnet, dass** die Krümmungen der Drähte (6) in Ebenen (16) verlaufen, die parallel zur Längsachse (12) aber in einer Distanz (17) zur Längsachse angeordnet sind.

6. Marknagel nach einem der Ansprüche 1 bis 5, **dadurch gekennzeichnet, dass** er aus Metall, beispielsweise aus einer Metalllegierung oder aus Titan besteht.

7. Marknagel nach einem der Ansprüche 1 bis 6, **dadurch gekennzeichnet, dass** an seinem Befestigungskopf (1) ein Zielbügel (26) für Querbohrungen (2) anbringbar ist.

8. Marknagel nach einem der Ansprüche 1 bis 7, **dadurch gekennzeichnet, dass** sein Schaft (19) eine Krümmung (22) in Längsrichtung aufweist.

9. Marknagel nach einem der Ansprüche 1 bis 7, **dadurch gekennzeichnet, dass** sein Schaft in einer vorgegebenen Zone (28) elastisch um einen Winkel grösser 2° verbiegbar ist.

10. Marknagel nach Anspruch 9, **dadurch gekennzeichnet, dass** die vorgegebene Zone (28) als ein- oder mehrgängige Wendel ausgeführt ist, welche als Zugfeder zur Aufrechterhaltung einer Kompressionskraft verwendbar ist.

## Claims

1. Medullar nail, preferably for the humerus, comprising a securing head (1) for a hammering-in and target device (24), with the medullar nail having at least one transverse bore (2) adjoining the securing head (1), and comprising an entry tip (3) and radially extensible anchoring elements (4) in their vicinity, wherein the anchoring elements (4) are combined in a claw (5) of curved wires (6), with the wires (6) being elastically deformed inside the medullar nail in a non-extended state and springing out radially and assuming their original curved shape with an axial advance of the claw, and wherein guiding grooves (7) are provided at the inner side of the medullar nail which extend in the longitudinal direction and which in each case have a radial aperture (8) in order to guide tips (9) of the deformed wires (6') up to their radial emergence, **characterized in that** the apertures have the width (10) of the guiding groove (7) and taper in the direction of the entry tip (3) down to the width (11) of a wire (6) in order to give the axially advanced, and radially emergent wires (6) support in the axial direction and against a rotation about the longitudinal axis (12).

2. Medullar nail in accordance with claim 1, **characterized in that** the claw (5) has a foot (13) which is axially displaceable via a setting screw (14).

3. Medullar nail in accordance with claim 1 or claim 2, **characterized in that** a rotational security is provided at the claw.

4. Medullar nail in accordance with any one of the claims 1 to 3, **characterized in that** at least three curved wires (6) form the claw (5).

5. Medullar nail in accordance with any one of the claims 1 to 4, **characterized in that** the curvatures of the wires (6) extend in planes (16) which are arranged parallel to the longitudinal axis (12), but at a distance (17) from the longitudinal axis.

6. Medullar nail in accordance with any one of the claims 1 to 5, **characterized in that** it consists of metal, for example of a metal alloy or of titanium.

7. Medullar nail in accordance with any one of the claims 1 to 6, **characterized in that** a target hoop (18) for transverse bores (2) can be attached to its securing head (1).

8. Medullar nail in accordance with any one of the claims 1 to 7, **characterized in that** its shaft (19) has a curvature (22) in the longitudinal direction.

9. Medullar nail in accordance with any one of the claims 1 to 7, **characterized in that** its shaft can be bent elastically by an angle larger than 2° in a predetermined zone (28).

10. Medullar nail in accordance with claim 9, **characterized in that** the predetermined zone (28) is formed as a singly or multiply threaded helix which can be used as tension spring for maintaining a compression force.

## Revendications

1. Clou intramédullaire, de préférence pour l'humérus, comportant une tête de fixation (1) pour un dispositif d'enfoncement et de ciblage (24), le clou intramédullaire présentant au moins un perçage transversal (2) en raccordement à la tête de fixation (1), et comportant une pointe d'entrée (3) ainsi qu'à sa proximité des éléments d'ancrage (4) susceptibles d'être déployés radialement, les éléments d'ancrage (4) étant rassemblés dans une griffe (5) en fils métalliques (6) recourbés, les fils métalliques (6) étant élastiquement déformés à l'intérieur du clou intramédullaire, et dans un état non sorti, ils jaillissant radialement avec un avancement axial de la griffe et retrouvent leur forme recourbée originale, et sur la face intérieure du clou intramédullaire sont ménagées des rainures de guidage (7) s'étendant en direction longitudinale, qui présentent chacune une percée radiale (8) pour guider jusqu'à leur sortie radiale les pointes (9) des fils métalliques (6') déformés,
**caractérisé en ce que** les percées ont la largeur (10) de la rainure de guidage (7) et se rétrécissent en direction de la pointe d'entrée (3) jusqu'à la largeur (11) d'un fil métallique (6) pour donner aux fils métalliques (6) avancés axialement et sortis radialement un soutien en direction axiale et à l'encontre d'une rotation autour de l'axe longitudinal (12).

2. Clou intramédullaire selon la revendication 1, **caractérisé en ce que** la griffe (5) présente un pied (13) qui peut être déplacé axialement via une vis de réglage (14).

3. Clou intramédullaire selon la revendication 1 ou 2, **caractérisé en ce qu'**une sécurité antirotation est montée sur la griffe.

4. Clou intramédullaire selon l'une des revendications 1 à 3, **caractérisé en ce qu'**au moins trois fils métalliques (6) recourbés forment la griffe (5).

5. Clou intramédullaire selon l'une des revendications 1 à 4, **caractérisé en ce que** les courbures des fils métalliques (6) s'étendent dans des plans (16) qui sont agencés parallèlement à l'axe longitudinal (12), mais à une distance (17) de l'axe longitudinal.

6. Clou intramédullaire selon l'une des revendications 1 à 5, **caractérisé en ce qu'**il est en métal, par exemple en un alliage métallique ou en titane.

7. Clou intramédullaire selon l'une des revendications 1 à 6, **caractérisé en ce qu'**un étrier de ciblage (26) pour des perçages transversaux (2) peut être monté sur sa tête de fixation (1).

8. Clou intramédullaire selon l'une des revendications 1 à 7, **caractérisé en ce que** son fût (19) présente une courbure (22) en direction longitudinale.

9. Clou intramédullaire selon l'une des revendications 1 à 7, **caractérisé en ce que** dans une zone prédéterminée (28) son fût (19) peut être plié élastiquement autour d'un angle supérieur à 2°.

10. Clou intramédullaire selon la revendication 9, **caractérisé en ce que** la zone prédéterminée (28) est réalisée sous forme d'une hélice à un ou plusieurs pas qui peut être utilisée en tant que ressort de traction pour maintenir une force de compression.
